# EUROPÄISCHE PATENTANMELDUNG

(11) **EP 1 764 129 A1**
(43) Veröffentlichungstag der Anmeldung: **21.03.2007**
(21) Anmeldenummer: 06017451.3
(22) Anmeldetag: 22.08.2006
(51) Int. Cl.: A61M 37/00, A61H 15/00, A61B 17/20

(54) **Vorrichtung zum Einbringen eines Wirkstoffs in die Haut**

(30) Priorität: 14.09.2005 DE 102005043929
(71) Anmelder: Liebl, Horst, 67860 Zelsheim/Elsass (FR)
(72) Erfinder: Liebl, Horst, 67860 Zelsheim/Elsass (FR)
(74) Vertreter: Tomerius, Isabel

(57) **Zusammenfassung**

Vorrichtung (1) zum Einbringen eines Wirkstoffs in die Haut. Die Vorrichtung umfasst mindestens zwei, um ihre Längsachse (7,7') drehbar gelagerte Walzen (3,3'), auf deren Außenumfangsfläche (2,2') eine Vielzahl von Nadeln (15) radial nach außen vorstehen. Um eine großflächige Anwendung der Vorrichtung zu erleichtern, sind die Walzen parallel zueinander und seitlich derart gegeneinander versetzt angeordnet, dass bei einer Projektion einer der Walzen in der Weise, dass ihre senkrecht zur Achsrichtung projizierte Längsachse auf der Längsachse der parallel angeordneten Walze zu liegen kommt, die Bereiche der Außenumfangflächen beider Walzen, in denen die Nadeln nach außen vorstehen, sich in Axialrichtung nicht überschneiden.

## Beschreibung

Die Erfindung betrifft eine Vorrichtung zum Einbringen eines Wirkstoffes in die Haut mit um ihre Längsachse drehbar gelagerten Walzen, auf deren Außenumfangsfläche eine Vielzahl von Nadeln, die zumindest in einem über die Außenumfangsfläche vorstehenden Spitzenbereich nach außen spitz zulaufend ausgebildet sind, radial nach außen vorsteht.

Beim Aufbau der Haut unterscheidet man grundsätzlich zwischen der Epidermis und der tiefergelegenen Dermis. Die etwa 0,03 bis 0,1 mm dicke Epidermis wird in der Regel in fünf weitere Hautschichten unterteilt und setzt sich überwiegend aus abgestorbenen Hornzellen zusammen. Dabei werden vom Körper kontinuierlich neue Hornzellen gebildet, sodass sich die Epidermis ständig erneuert. Der grundsätzlich erstrebenswerten natürlichen Barrierefunktion der Epidermis, wie z. B. der Abschirmung der unteren Hautschichten gegen äußere bakterielle Einflüsse, physikalische Noxen usw., steht der Nachteil einer stark herabgesetzten Penetrierbarkeit von topisch applizierten Wirkstoffen gegenüber.

Insbesondere in der Dermatologie und der Kosmetik ist eine oberflächliche Applikation von Wirkstoffen, in der Regel in Form von wirkstoffhaltigen streichfähigen Dermatika wie Salben und Cremes, zum Einbringen von Wirkstoffen in die Haut sehr weit verbreitet. Die darin enthaltenen Wirkstoffe können zum Beispiel der Hautalterung entgegenwirken.

Eine Alterung der Haut äußert sich unter anderem durch einen Verlust an Transparenz, an Feuchtigkeit und an Straffheit. Diese Symptome lassen sich auf die im zunehmenden Alter natürliche Abnahme der Dicke der Hautschichten, den Abbau vom Fettgewebe in tieferen Hautschichten, aber auch auf ein Schrumpfen und eine Verhärtung von Kollagenfasern zurückführen.

Es ist bekannt, dass eine Vielzahl von Wirkstoffen die revitalisierenden Prozesse, wie z. B. die Bildung neuen Kollagengewebes, in der tiefergelegenen Dermis anregen und fördern. Damit ein auf die Haut applizierter Wirkstoff jedoch an den Wirkort, respektive in die Dermis, gelangen kann, muss die Epidermis, die in ihrer natürlichen Funktion das Vordringen von für die Haut nützlichen Substanzen behindert, überwunden werden.

Klinische Studien haben gezeigt, dass auf herkömmliche Weise auf die Haut aufgetragene Wirkstoffe in der Regel zu mehr als 99,7% in der Epidermis verbleiben. Entsprechend durchdringen lediglich maximal 0,3% der aktiven Substanzen die Epidermis und gelangen in die Dermis, wo sie ihre gewünschte Wirkung entfalten können.

Um die Wirkstoffkonzentration in der Dermis zu erhöhen, wird häufig der Wirkstoffanteil in den verwendeten Salben, Cremes usw. heraufgesetzt. Allerdings ruft dieses Verfahren eine in der Regel ungewollte, dramatisch erhöhte Wirkstoffakkumulation in der Epidermis hervor, wodurch Nebenwirkungen, die bis hin zu einer Zerstörung der Epidermisschichten reichen können, auftreten. Ein Verlust des äußeren Schutzpanzers der Haut jedoch hat gravierende Folgen. So zeigt eine derartig betroffene Hautoberfläche nahezu immer eine Überempfindlichkeit gegenüber äußeren Einflüssen wie UV-Strahlung, Trockenheit etc.

Alternativ werden topischen Formulierungen Zusatzstoffe wie Liposomen, Säuren etc. zugesetzt. Neben der technologisch häufig sehr aufwändigen Herstellung entsprechender Topika führen diese Zusatzstoffe in der Regel ebenfalls zu einer Zerstörung der Epidermisschicht, sodass auch hier die oben genannten Nachteile zu beobachten sind.

Eine weitere Möglichkeit besteht in der mechanischen Entfernung der Epidermis, um den Wirkstoffen den Zugang zur Dermis zu erleichtern. Dazu werden die oberen Hautschichten mit Diamantfräsen weggeraspelt, mit Lasern verdampft etc. Diese drastischen Alternativen sind in der Regel für die behandelte Person äußerst unangenehm und schmerzhaft, so dass hier eine begleitende analgetische Therapie sowie eine häufig sehr langwierige Wundpflege nötig sind. Zudem resultiert auch diese Vorgehensweise in einem vorübergehenden Verlust der natürlichen Schutzfunktion der Haut. Als Folge ist auch eine derartig behandelte Hautoberfläche außerordentlich empfindlich gegenüber externen Faktoren.

Mit dem in der DE 10063634 A1 beschriebenen Nadelroller entwickelte der Anmelder bereits eine Methode, mit der die Wirkstoffpenetration in die Dermis um mehr als das 40-fache im Vergleich zu unbehandelter Haut erhöht werden kann, ohne dabei die Hautoberfläche nachhaltig zu beschädigen. Darüber hinaus ist die Behandlung für die betreffende Person praktisch schmerzfrei. In seltenen Fällen können leichte Hautirritationen beobachtet werden, die aber bereits nach wenigen Tagen wieder vollständig verschwinden. Die natürliche Schutzfunktion der Epidermis hingegen steht nahezu unmittelbar nach der Behandlung wieder vollständig zur Verfügung, da sich die durch die Nadeln des Nadelrollers auf der Haut hervorgerufenen Einstichkanäle in der Regel nach wenigen Minuten wieder schließen. Ferner ermöglicht der Nadelroller eine definierte und reproduzierbare Eindringtiefe der Nadeln in die Haut zu gewährleisten, was einen beträchtlichen Zugewinn an Bedienungskomfort und -sicherheit sowohl für die behandelnde als auch für die behandelte Person bedeutet.

Zur Behandlung der Hautoberfläche wird die drehbar gelagerte Walze des Nadelrollers manuell über ein Hautareal bewegt, auf das entweder vorher oder nachher ein Wirkstoff bzw. ein den Wirkstoff enthaltendes Gemisch aufgetragen wird. Um den in der DE 10063634 A1 beschriebenen Nadelroller über die Haut zu führen, ist die Walze an einer Gabel befestigt, an der wiederum ein Handgriff angebracht ist. Über die Außenumfangsfläche der Walze steht eine Vielzahl von Nadeln radial nach außen vor, die in die Haut eindringen und somit winzige Kanäle öffnen, durch welche der applizierte Wirkstoff die Epidermis passieren und letztendlich in tiefere Hautschichten vordringen kann. Die Kanäle bleiben dabei solange für einen Wirkstoff passierbar, dass weitere Wirkstoffe sukzessive in die Haut eindringen können. Unterstützende Maßnahmen, wie ein Massieren der Haut oder ähnliches, können dabei die Wirkstoffpenetration weiter fördern. Besonders vorteilhaft an dem Nadelroller ist zudem, dass, nachdem die Nadeln bereits vollständig mit der über die Außenumfangsfläche der Walze vorstehenden Länge in die Haut eingedrungen sind, die Walzen mit der Walzenaußenfläche auf der Haut aufliegen. Dadurch können die Nadeln ausschließlich nur bis zu einer vorgegebenen Maximaltiefe in die Hautoberfläche eindringen, unabhängig davon, wie hoch der auf die Walze ausgeübte Druck ist.

Die Nadeln sind, um ein Eindringen in die Haut zu erleichtern, zumindest in einem über die Außenumfangsfläche vorstehenden Spitzenbereich nach außen spitz zulaufend ausgebildet. Ferner sind die Nadeln regelmäßig und gleichmäßig auf den Walzen verteilt, wodurch eine gezielte und reproduzierbare Steuerung der Anzahl der Einstichkanäle über die Häufigkeit der Bewegungen des Nadelrollers über die Haut ermöglicht wird. Durch eine Variation der Einstichanzahl pro Flächeneinheit auf der Haut kann so ebenfalls direkt Einfluss auf die Wirkstoffpenetration genommen werden.

Klinischen Tests zufolge ist der Nadelroller insbesondere zur Behandlung der oft großflächig auftretenden Zellulite ("Orangenhaut") in Verbindung mit gewissen Wirkstoffen geeignet und kann hier in vergleichsweise kurzer Zeit deutliche Behandlungserfolge erzielen.

Dabei ist jedoch die Behandlung einer großen Hautoberfläche, z. B. eines Oberschenkels, durch den Nadelroller der DE 10063634 A1 auf Grund einer Walzenbreite von 1 bis 2 cm vergleichsweise zeitintensiv, da die resultierenden Streifenbreiten der von den Nadeln durchdrungenen Bereiche entsprechend auf eine Breite von 1 bis 2 cm begrenzt sind. Für eine großflächige Anwendung ist der Nadelroller der DE 10063634 A1 somit weniger geeignet.

Angesichts dessen ist es die Aufgabe der Erfindung, eine Vorrichtung anzugeben, die die gleichmäßige Einbringung eines Wirkstoffes in großflächige Hautbereiche in kurzer Zeit ermöglicht. Darüber hinaus muss die Vorrichtung so angelegt sein, dass eine sichere und zuverlässige Behandlung der Hautoberfläche gewährleistet ist.

Ausgehend von der DE 10063634 A1 besteht zur Lösung der Aufgabe die Möglichkeit, die beschriebenen Nachteile durch eine Vergrößerung der axialen Walzenlänge des Nadelrollers zu überwinden. Problematisch ist an diesem Lösungsansatz, dass die resultierende sehr breite Walze während der Anwendung auf der Haut beträchtlichen Drücken bzw. Biegemomenten ausgesetzt ist. Es besteht daher ein erhöhtes Risiko, dass die Walze entlang ihrer Achse verbiegt, wodurch sie nicht mehr gleichmäßig auf der Haut entlanggerollt werden kann. Verbiegt die Walze noch stärker besteht sogar die Gefahr, dass die Walze aus der Gabel herausgedrückt wird. Ferner lockert eine permanente Verbiegung der Walze die Verklebungen zwischen den einzelnen Walzenscheiben der DE 10063634 A1, so dass die Nadeln ihre definierte Positionierung verlieren und nicht mehr in vorbestimmter Art und Weise in die Hautoberfläche eindringen. Durch die gelockerte Verbindung zwischen den Walzenscheiben ist die Wahrscheinlichkeit deutlich erhöht, dass die Walze während einer Behandlung zerbricht. Zusammenfassend besteht daher bei einer derartig verbreiterten Walze ein erhebliches Verletzungsrisiko durch zum Beispiel Nadeln, die in der Haut stecken bleiben, die tiefer als gewünscht in die Haut eindringen, die undefinierte Schnitte und Risse auf der Haut verursachen. Ferner kann ein plötzliches Herausrutschen der Walze aus der Gabel oder gar ein Brechen der Walze weitere erhebliche Verletzungen auslösen.

Um das aufgezeigte erhöhte Verletzungspotential einer entsprechend axial verlängerten Walze effektiv zu senken, steigen die konstruktiven Anforderungen unverhältnismäßig stark an. So ist unter anderem eine vergleichsweise aufwändige und kostenintensive passgenaue Achse erforderlich, um die Walzenscheiben zusätzlich seitlich stabilisieren zu können. Ferner muss die Gabel bedeutend massiver angelegt sein, um die resultierenden Kräfte ohne Deformation aufnehmen zu können, eine exakte Führung der Walze zu ermöglichen und ein unbeabsichtigtes Herausspringen der Walze aus der Gabel zu verhindern. Die dargestellten Sonderanforderungen an die technische Realisation eines solchen verbreiterten Nadelrollers hätte insgesamt eine dramatische Zunahme der Produktionskosten zur Folge.

Die Lösung der Aufgabe gelingt vielmehr mit der Vorrichtung gemäß Anspruch 1. Bevorzugte Ausführungsformen sind den Unteransprüchen zu entnehmen.

In einem ersten Aspekt betrifft die Erfindung eine Vorrichtung zum Einbringen eines Wirkstoffes in die Haut, die mindestens zwei mit Nadeln versehene Walzen umfasst, wobei sich der grundsätzliche Aufbau der Nadeln und Walzen an dem in der DE 10063634 A1 offenbarten Nadelroller orientiert. Die Walzen einer erfindungsgemäßen Vorrichtung sind parallel zueinander angeordnet, um ein kontinuierliches Rollen auf der Hautoberfläche zu ermöglichen. Beim Entlangrollen der Walzen auf der Haut dringen die über die Außenumfangsflächen der Walzen vorstehenden Nadeln in die Epidermis ein und hinterlassen streifenförmige und in der Regel nicht sichtbare Flächen mit winzigen Einstichen auf der Hautoberfläche. Diese streifenförmigen Flächen werden im Folgenden Walzstreifen genannt. Jede der mindestens zwei Walzen hinterlässt auf der Haut jeweils einen Walzstreifen, dessen Breite der Länge der axialen Außenkante der korrespondierenden Walze entspricht. Dabei sind die Walzen nicht nur parallel zueinander, sondern auch seitlich derart gegeneinander versetzt angeordnet, dass bei einer Projektion einer der Walzen in der Weise, dass ihre senkrecht zur Achsrichtung projizierte Längsachse auf der Längsachse der parallel angeordneten Walze zu liegen kommt, sich die Bereiche der Außenumfangsflächen beider Walzen, in denen die Nadeln nach außen vorstehen, in Axialrichtung nicht überschneiden. Vorteilhaft an dieser Anordnung ist, dass sich durch den in Rollrichtung seitlichen Versatz der Walzen bei einem Entlangrollen der Vorrichtung auf der Haut die einzelnen Walzstreifen nicht überschneiden, sondern parallel nebeneinander verlaufen. Dadurch kann mit den verfügbaren Walzen die maximale Gesamtwalzbreite der Vorrichtung erzielt werden. Dabei sind die Walzen in ihrem grundsätzlichen Aufbau mit den bereits bewährten Walzen aus der DE 10063634 A1 vergleichbar.

Bevorzugt sind die Walzen seitlich derart zueinander versetzt angeordnet, dass sich die einzelnen Walzstreifen zu einer im Wesentlichen zusammenhängenden Fläche ergänzen. Diese besondere Anordnung entspricht in ihrer Wirkung einer einzelnen sehr breiten Walze, wobei jedoch die mit einer stark verbreiterten Walze assoziierten, oben dargelegten konstruktiven Nachteile nicht auftreten. Im Vergleich zu dem in der DE 10063634 A1 beschriebenen Nadelroller hingegen wird die aufzuwendende Zeit deutlich reduziert, um ein definiertes Hautareal mit der Vorrichtung gleichmäßig abzurollen und mit Einstichkanälen zu versehen.

Weist die Vorrichtung mehr als zwei Walzen auf, werden diese bevorzugt entlang nur zweier Längsachse angeordnet, um eine kompakte Bauart der Vorrichtung zu erzielen. Ganz besonders bevorzugt werden die mehr als zwei Walzen alternierend in der Art und Weise angeordnet, dass die Längsachsen direkt benachbarter Walzen parallel zueinander und koaxial mit der Längsachse der darauf folgenden Walze verlaufen. Somit ist jede Walze, die über zwei an der Vorrichtung angebrachte Walzen hinaus geht, koaxial zu einer in einer Axialrichtung übernächsten Walze angeordnet. Dadurch kann eine besonders kompakte Ausführungsform der Vorrichtung erreicht werden, weil die Walzen sehr platzsparend angeordnet werden können und trotzdem die Walzstreifen sehr nah beieinander liegen bzw. sich zu einer nahezu übergangslosen Gesamtfläche ergänzen.

Als besonders vorteilhaft hat sich eine Ausführungsform mit insgesamt drei Walzen herausgestellt, wobei die Walzen V-förmig zueinander angeordnet sind. Die Längsachse einer Walze verläuft dabei parallel zu der Längsachse der beiden anderen Walzen, die koaxial zueinander angeordnet sind. Ferner ist die eine parallel angeordnete Walze in Axialrichtung zwischen den beiden anderen Walzen angebracht. Da die Breite einer einzelnen Walze vorzugsweise in einem Bereich von 1 bis 3 cm und insbesondere bei 2 cm liegt, weist der Gesamtwalzstreifen bei der bevorzugten abstandsfreien Anordnung der Walzen eine Gesamtbreite von 3 bis 9 cm und insbesondere 6 cm auf. Es hat sich gezeigt, dass mit diesen bevorzugten Abmessungen der Walzen Vorrichtungen erhalten werden, die sich einerseits in ihrer Kompaktheit durch eine besonders gute Handhabbarkeit auszeichnen, da sie bequem in einer Hand zu halten sind. Andererseits wird die nötige Behandlungszeit im Vergleich zum Nadelroller der DE 10063634 A1 immerhin um den Faktor drei gesenkt. Darüber hinaus kippelt die Vorrichtung durch die V-förmige Anordnung der Walzen nicht, sondern steht stabil auf der Hautoberfläche. Diese Eigenschaft erlaubt einerseits eine praktisch wackelfreie Führung der Vorrichtung auf der Haut, andererseits kann die Vorrichtung auch beiseite gestellt werden, ohne das sie umkippt und die Gefahr eines unbeabsichtigten Hineingreifens in die Nadelwalzen besteht.

Darüber hinaus ist es von Vorteil, alle Walzen der Vorrichtung baugleich auszuführen, wodurch die einzelnen Walzen auf die gleiche Art und Weise hergestellt werden können und die Produktionskosten der Vorrichtung erheblich gesenkt werden.

Um eine homogene Penetrationsverbesserung über den gesamten zu behandelnden Hautbereich zu gewährleisten, empfiehlt es sich, die Nadeln gleichmäßig über die Außenumfangsfläche jeder einzelnen Walze, aber auch gleichmäßig über die Gesamtheit der Walzen zu verteilen. Dazu sind die Walzen der Vorrichtung, wie bereits aus der DE 10063634 A1 bekannt ist, zweckmäßig jeweils aus mehreren parallelen Scheiben aufgebaut, zwischen denen die Nadeln in regelmäßigen Abständen eingebettet sind. Vorteilhafterweise weisen die Scheiben dazu Vertiefungen auf, die sich wenigstens auf einer der Seiten der Scheiben in radialer Richtung nach außen erstrecken. Darüber hinaus ist es von Vorteil, wenn die Länge, mit der die Nadeln über die Außenumfangsflächen der Walzen hervorstehen, in definierten Grenzen liegen, damit alle Nadeln gleich tief in die Hautoberfläche eindringen. Um ein Verrutschen der Nadeln in das Innere der Walze hinein zu verhindern, ist dazu zweckmäßigerweise für jede der Nadeln ein Anschlag am inneren Ende der Vertiefungen vorgesehen.

Bevorzugt stehen die Nadeln mit gleicher Länge und insbesondere mit einer Länge von 0,12 bis 1,5 mm über die Außenumfangsflächen der Walzen vor. Um ein Eindringen der Nadeln in die Haut zu erleichtern, sind diese zweckmäßig in dem über die Außenumfangsfläche der Walzen vorstehenden Spitzenbereich nach außen hin spitz zulaufend ausgebildet. Dabei ist es von Vorteil, wenn runde, atraumatische Nadeln verwendet werden, die beim Eindringen der Nadeln in die Haut die Hornplättchen der Epidermis auseinander schieben. Nicht bevorzugt sind dagegen die in der Medizin verbreiteten traumatisch wirkenden Trokare, die das Gewebe eher zerschneiden, womit die Epidermis eine bedeutend längere Regenerationsphase braucht, um ihrer Schutzfunktion wieder in vollem Umfang nachzukommen. Zweckmäßig sind die Nadelspitzen so dünn wie möglich, ohne dass jedoch die Gefahr eines Abbrechens der Nadeln besteht. Dabei hängt der realisierbare optimale Durchmesser der Nadelspitzen größtenteils von dem verwendeten Material ab. Ferner kann der Durchmesser der Nadelspitzen auf die Art der Anwendung hin optimiert werden. Als vorteilhaft haben sich Nadelspitzen mit einem Durchmesser im Spitzenbereich von 0,05 mm bis 0,1 mm und ganz besonders vorteilhaft von 0,08 mm erwiesen. Außerhalb des Spitzenbereiches haben die Nadeln zweckmäßiger Weise einen Durchmesser von 0,15 bis 0,3 mm. Vorzugsweise bestehen die Nadeln der erfindungsgemäßen Vorrichtung aus Metall und insbesondere aus Edelstahl. Die Spitzen der Nadeln werden üblicherweise zugeschliffen und anschließend elektropoliert.

Bei einer aus mehreren Scheiben aufgebauten Walze werden zunächst die Nadeln vorzugsweise in die auf den Scheiben vorgesehenen Vertiefungen eingeklebt, um die Nadeln in ihrer Lage zusätzlich zu stabilisieren. Anschließend werden die Scheiben parallel zueinander miteinander verbunden. Auch hierzu kann ein Klebstoff verwendet werden. Damit die Scheiben die gewünschte Orientierung zueinander aufweisen, ist es im Rahmen der Erfindung besonders bevorzugt, auf wenigstens einer der Oberflächen einer Scheibe wenigstens einen Zentriervorsprung vorzusehen. Diesem wenigstens einen Zentriervorsprung entspricht eine entsprechende Zentriervertiefung auf der Oberfläche der benachbarten Scheibe. Besonders zweckmäßig ist es, wenn der Zentriervorsprung gleichzeitig als hinterer Anschlag für die Nadeln dient. Besonders bevorzugt sind solche Zentriervorsprünge, welche gleichzeitig zu einer Zentrierung benachbarter Scheiben und zur Einstellung eines vorgegebenen Winkelversatzes der Nadeln der benachbarten Scheiben zueinander führen. Geeignet hierfür ist beispielsweise ein Vorsprung in Form eines Zahnkranzes, welcher im Zentrum der Scheibe vorspringt. Eine entsprechende Vertiefung in Form des Zahnkranzes ist in einer benachbarten Scheibe ausgebildet und nimmt den Vorsprung der anderen Scheibe formschlüssig auf.

Die Anordnung benachbarter Scheiben einer Walze zueinander kann dabei so erfolgen, dass die Nadeln aller benachbarter Scheiben auf Linien liegen, welche parallel zur Walzenlängsachse verlaufen. Alternativ ist es möglich, die Nadeln benachbarter Scheiben auf Lücke anzuordnen. Vorteilhafterweise wird der seitliche Versatz der Walzen zueinander so gewählt, dass sich das Anordnungsmuster der Nadeln auf einer Walze nahezu übergangslos auf den in Axialrichtung benachbarten Walzen fortsetzt.

Die Anzahl der Nadeln auf einer Walze vorgegebener Größe kann einerseits über die Anzahl der Scheiben - also über die Scheibendicke - sowie die Anzahl der Nadeln pro Scheibe gesteuert werden. Es hat sich gezeigt, dass sich eine allzu dichte Besetzung der Walze mit Nadeln negativ auswirken kann, da eine Art "Fakirkissen-Effekt" auftritt und die Nadeln nur noch schwer in die Haut eindringen können. Geeignete Bestückungszahlen werden beispielsweise erreicht, wenn die Nadeln pro Scheibe auf Geraden angeordnet werden, welche vom Mittelpunkt der Scheibe aus einen Winkel von 15 oder 30° bilden. Dies entspricht 24 bzw. 12 Nadeln pro Scheibe. Geeignete Scheibendicken liegen beispielsweise bei 2 bis 3 mm, insbesondere bei 2,5 mm.

Die Anzahl der verwendeten Scheiben richtet sich zweckmäßig nach der beabsichtigten Verwendung der erfindungsgemäßen Vorrichtung. Je nach Größe der zu bearbeitenden Hautpartie können beispielsweise 2 bis 3 Scheiben pro Walze ausreichen, es können aber auch 10 oder mehr Scheiben verwendet werden. Grenzen liegen hier lediglich zwingend erforderlichen Aufrechterhaltung der Biegefestigkeit bzw. der Stabilität der erhaltenen Einzelwalze. Typischerweise werden die Einzelwalzen aus 7 Scheiben aufgebaut. Als stirnseitige Abschlüsse der Scheibenanordnung können Endscheiben verwendet werden, die beispielsweise zu einem glatten axialen Abschluss der so entstandenen Walze führen.

Der Durchmesser einer Walze bzw. der sie bildenden Scheiben kann ebenfalls über einen breiten Bereich variiert werden. Durch Variation der Größe des Durchmessers kann ferner Einfluss auf den Abstand benachbarter Nadeln ausgeübt werden. Außerdem ist es möglich, durch Variation der Größe des Durchmessers die Länge des Überstands der Nadeln über den Außenumfang einzustellen und damit die Eindringtiefe der Nadeln zu steuern. Auf diese Weise können ohne Änderung der Nadellänge Vorrichtungen mit unterschiedlicher Eindringtiefe der Nadeln hergestellt werden. Umgekehrt ist es möglich, den Durchmesser der Walze oder der die Walze bildenden Scheiben konstant zu halten und statt dessen die Nadellänge zu variieren und auf diese Weise Vorrichtungen mit unterschiedlicher Eindringtiefe der Nadeln zu erhalten. Beispielhaft können Walzendurchmesser von 1 bis 5 cm genannt werden. Besonders geeignet sind Walzen mit einem Durchmesser von etwa 2 bis 3 cm.

Damit die mindestens zwei Walzen einer erfindungsgemäßen Vorrichtung bequem und sicher über die Hautoberfläche geführt werden können, weist die Vorrichtung bevorzugt ein Gehäuse auf, das aus einem oder mehreren Teilen aufgebaut sein kann. Dabei schirmt das Gehäuse die Walzen auf der hautabgewandten Seite nach oben hin ab, so dass die Bedienperson vor einem unbeabsichtigtem Berühren der Nadelspitzen der Hand bzw. Handfläche geschützt wird. Darüber hinaus wird die hygienische Sicherheit der Bedienungsperson erhöht. Dies ist insbesondere dann von Vorteil, wenn die behandelnde Person nicht mit der behandelten Person identisch ist. Vorzugsweise wird das Gehäuse so konstruiert, dass die Bedienperson mit der Handinnenfläche auf der Gehäuseoberseite aufliegt bzw. das Gehäuse mit dem Daumen und einem weiteren Finger zusätzlich seitlich umgreifen kann. Diese bevorzugte Ausführungsform kann sehr sicher festgehalten werden und erlaubt daher eine kontrollierte Führung der Walzen über die Hautoberfläche. Ganz besonders bevorzugt ist es, die grundsätzliche Gehäuseform an die Form einer Computermaus anzulehnen. Ein solches anatomisch geformtes Gehäuse stellt insbesondere für die Personen eine erhebliche Erleichterung dar, die täglich bzw. über längere Zeiträume die Vorrichtung bedienen. Zudem ist diese Form ausgeprägt handlich und kompakt. In der Regel ist die Oberseite eines solchen Gehäuses im Mittelbereich leicht nach oben gewölbt und fällt in dem der Vorderseite zugewandten Bereich flacher nach vorne ab als in Richtung der gegenüberliegenden hinteren Kante und der beiden Seitenbereiche.

Es ist zweckmäßig, die Walzen mit dem Gehäuse so zu verbinden, dass zumindest der Spitzenbereich der Nadeln über eine durch die Unterkante des Gehäuses aufgespannte Ebene vorsteht, damit die Nadeln überhaupt in die Hautoberfläche eindringen können. Um allerdings ein unproblematisches Rollen der Vorrichtung auf der Haut zu ermöglichen, stehen die Unterkanten der Außenumfangsflächen der Walzen vorzugsweise mit einer Höhe von 2 mm bis 8 mm und insbesondere 5 mm über die Unterkante des Gehäuses hervor.

Zur drehbaren Lagerung der Walzen sind prinzipiell alle üblichen Anordnungen denkbar, die eine freie Drehbarkeit der Walzen um ihre Längsachsen ermöglichen. So können axiale Verlängerungen mit in die Endscheiben integriert werden, die schließlich von zum Beispiel am Gehäuse angebrachten Lagern aufgenommen werden. Alternativ können die Walzen auch seitliche Einstülpungen aufweisen, in die am Gehäuse angebrachte geeignete Ausstülpungen hineinragen. Vorteilhaft ist indes die Verwendung von Achsen, die die Walzen entlang ihrer Längsachse durchlaufen. Solche Achsen verleihen den Walzen zusätzliche Stabilität bzw. eine erhöhte Biegefestigkeit und entlasten zudem die Verbindungen der einzelnen Walzenscheiben untereinander. Dabei ist es möglich, dass mehrere Walzen auf ein und derselben Achse angebracht sind. Bevorzugt weist allerdings jede Walze eine separate Achse auf, um die technischen Anforderungen an die Achsen zu reduzieren und die auf jeder einzelnen Achse lastenden Kräfte möglichst gering zu halten. Um eine Achse durch die Walzen führen zu können, weisen die einzelnen Walzenscheiben in ihrem Mittelpunkt eine Durchgangsbohrung auf, deren Durchmesser zweckmäßigerweise im Wesentlichen dem Durchmesser der Achsen entspricht. Grundsätzlich ist der Achsendurchmesser variabel, vorzugsweise liegt er allerdings zwischen 1 und 3 mm und insbesondere bei 2 mm. Allgemein eignen sich zur Herstellung der Achsen alle Materialien, die die praktischen Anforderungen hinsichtlich Stabilität und Biegefestigkeit erfüllen. So ist es zum Beispiel denkbar, Kunststoffachsen oder Achsen aus einem geeigneten Metall zu verwenden. Dabei haben sich speziell Edelstahlachsen als besonders geeignet erwiesen.

Um die Achsen mit dem Gehäuse zu verbinden, ist es besonders vorteilhaft, wenn die Achsen zu beiden axialen Seiten der Walze überstehen. Die resultierenden Achsenüberstände können nunmehr von üblichen Lagern aufgenommen werden, die dazu am Gehäuse angebracht sind. So ist es zum Beispiel denkbar, dass am Gehäuserand Auskerbungen oder Ausschnitte angebracht sind, in die die Achsenüberstände einrasten. Alternativ können die Achsen durch entsprechende Lochungen am Gehäuse geführt werden. Ferner ist es möglich, die Achsen zwischen aufeinanderliegenden Gehäuseteilen zu lagern. Dazu können zusätzlich Rinnen und Auskerbungen an den Gehäuseteilen vorgesehen sein. Grundsätzlich können die Achsen drehbar oder fest mit dem Gehäuse verbunden werden. Werden die Achsen drehbar am Gehäuse gelagert, sind die Walzen zweckmäßig fest mit den Achsen verbunden. Werden die Achsen hingegen fest am Gehäuse angebracht, drehen sich die Walzen jeweils um die die betreffende Walze durchlaufende Achse.

Zur Befestigung der Walzen am Gehäuse haben sich allerdings paarweise angeordnete, in Richtung der der Haut zugewandten Seite vorstehende und zueinander beabstandete Streben als besonders geeignet erwiesen, da diese zuverlässig ein axiales Spiel der Walze begrenzen und die resultierenden Kräfte optimal ins Gehäuse ableiten. Dabei ist ein Strebenpaar in der Regel um eine Walzenbreite voneinander beabstandet. Zur Aufnahme der Achsenüberstände weist jedes Strebenpaar spezielle Aussparungen auf, in die die aus einer Walze axial herausragenden Achsenabschnitte der Walze eingefügt werden. Bevorzugt ist die Aussparung auf der der Haut zugewandten Stirnseite der Strebe angebracht, wodurch die Montage der Vorrichtung erleichtert wird. Um die Achse in ihrer Position horizontal zu sichern, hat die Aussparung vorteilhafterweise eine Breite, die im Wesentlichen dem Durchmesser des Achsenüberstandes entspricht. Die Streben sind in ihren Dimensionen so ausgelegt, dass sie den beim Rollen der Vorrichtung auf der Haut ausgeübten Druck aufnehmen können. Bevorzugt weisen die Streben dabei in Axialrichtung der Walzen eine Dicke von 1 mm bis 4 mm und insbesondere von 2 mm. Senkrecht zur Axialrichtung haben die Streben eine bevorzugte Breite von 5 mm bis 20 mm und ganz besonders von 10 mm. Vorteilhafterweise sind die Streben im Innenraum der Vorrichtung an der Oberseite des Gehäuses befestigt und verlaufen senkrecht in Richtung der Hautoberfläche. Dies ermöglicht eine besonders stabile Anordnung, da die Streben den resultierenden Druck optimal aufnehmen und weiterleiten können.

Bei einer besonderen Ausführungsform der Vorrichtung ist der Durchmesser der Achsenüberstände kleiner als der Durchmesser der übrigen Achse. In diesem Fall liegt der Achsendurchmesser des Achsenüberstandes bevorzugt in einem Bereich zwischen 0,5 mm und 2 mm und insbesondere bei 1 mm. Die Streben, deren Aussparungen eine Breite haben, die im Wesentlichen dem Achsendurchmesser des Achsenüberstandes entspricht, verhindern in dieser Ausführungsform ein seitliches Herausrutschen der Achsen aus den Aussparungen. Der Achsendurchmesser des Mittelteiles der Achse ist zu breit, um in die Aussparung der Strebe hineinzupassen.

Darüber hinaus empfiehlt es sich, die Achsen in den Aussparungen der Streben so zu befestigen, dass die Achsenüberstände weder beim Transport noch bei einer Benutzung der Vorrichtung aus den Aussparungen der Streben herausfallen. Dazu können die Aussparungen der Streben zum Beispiel so gestaltet sein, dass die Achsenüberstände in den Aussparungen einrasten. Allerdings besteht bei dieser speziellen Vorgehensweise die Gefahr, dass die Achsenüberstände beim Andrücken bis zum Einrasten verbiegen. Außerdem stehen über die gesamte Außenfläche der Walze spitze Nadeln über, wodurch ein kontrolliertes Andrücken weiter erschwert wird. Es hat sich daher als vorteilhaft erwiesen, die Achsenüberstände in den Aussparungen durch Fixiermittel zu sichern. Diese werden, nachdem der Achsenüberstand in die Aussparung eingebracht wurde, in den verbliebenen Freiraum der Aussparung auf den Achsenüberstand geschoben. Damit das Fixiermittel nachhaltig fest mit der Strebe verbunden ist, wird es bevorzugt mit der Strebe verklebt. Liegen die Aussparungen ferner auf der der Haut zugewandten Seite der Strebe, werden die Fixiermittel aus Richtung der Hautoberfläche in die Aussparungen eingefügt. Beim Entlangrollen der erfindungsgemäßen Vorrichtung auf der Hautoberfläche üben die Achsenüberstände somit keinen Druck auf die Fixiermittel aus. Die Materialbeanspruchung der Befestigung des Fixiermittels ist daher bei dieser bevorzugten Positionierung der Aussparungen äußerst gering. Die Form der einzelnen Fixiermittel kann variieren. So können die beiden gegenüberliegenden Seiten des Fixiermittels, die im Kontakt mit den Seitenwänden der Aussparungen stehen, trapezförmig verlaufen. Der so erhaltene Keil fügt sich anschließend leicht in die Aussparung ein. In der Regel wird allerdings eine Quaderform mit einer Breite, die im Wesentlichen der Breite der Aussparung entspricht, bevorzugt, da diese, unabhängig wie weit das Fixiermittel in die Aussparung hineinragt, bis kurz vor die Achse geschoben werden kann.

Die einzelnen Fixiermittel sind in der Regel klein und unhandlich. Es empfiehlt sich daher, mehrere oder alle der benötigten Fixiermittel in einem gemeinsamen zusammenhängendem Bauteil wie zum Beispiel einer Leiste, einem Rahmen oder einem Gehäuseunterteil zu integrieren. Dadurch können alle benötigten Fixiermittel einer erfindungsgemäßen Vorrichtung in einem einzigen Arbeitsschritt mit dem entsprechenden Gehäuseteil hergestellt und mit diesem in die Aussparungen der Streben eingefügt werden, die dazu bevorzugt am korrespondierenden Gehäuseoberteil angebracht sind. Durch die übergangslose Verbindung der Fixiermittel zum Unterteil erlangt die Verbindung der Fixiermittel mit den Aussparungen zusätzlich eine erheblich höhere Stabilität.

Um ein gleichmäßiges Entlangrollen der Vorrichtung auf der Hautoberfläche zu erleichtern, ist es möglich, das Gehäuse nach unten mit einer vorzugsweise glatten Fläche so weit es geht zu verschließen. Dazu weist die Fläche einzelne Öffnungen auf, durch die die Walzen hindurchragen können. Diese Weiterbildung ist insbesondere dann von Vorteil, wenn die Walzen mit nur einem sehr geringen Abstand über die Unterseite des Gehäuses hervorstehen und daher bei einem Andrücken der Vorrichtung auf der Haut sehr schnell ein Teil der Unterkante des Gehäuses gegen die Hautoberfläche drückt, wodurch der Bewegungsfluss behindert wird. Es ist sinnvoll, die Öffnung so klein wie möglich und so groß wie nötig zu gestalten. So kann ein unbeabsichtigtes Einklemmen einer Hautfalte zwischen dem Rand der Öffnung und einer Walze, was für die behandelte Person ohne Zweifel sehr unangenehm wäre, nahezu ausgeschlossen werden. Eine Verschluss der Gehäuseunterseite wird besonders bevorzugt durch ein separates Unterteil hergestellt. Dazu wird das Unterteil, welches bereits eine glatte Außenfläche und die entsprechenden Öffnungen für die Walzen aufweist, an der Unterseite des Gehäuseoberteils angebracht.

Besteht das Gehäuse aus mehreren Teilen und insbesondere aus einem Oberteil und einem Unterteil, ist es sinnvoll, die Gehäuseteile so fest miteinander zu verbinden, dass das Gehäuse der Vorrichtung im Gebrauch eine hinreichend hohe Stabilität besitzt. Generell eignen sich hierzu die üblichen Verbindungsmethoden. So können die Gehäuseteile miteinander verspannt, verklebt oder verschweißt werden. Ferner ist es denkbar, die einzelnen Bestandteile des Gehäuses miteinander zu verschrauben, zu vernieten u.s.w.. Bevorzugt werden die Gehäuseteile allerdings mit gebräuchlichen Rastverbindungen aneinander befestigt. Dazu weist beispielsweise ein Gehäuseteil Auskerbungen auf, in die am benachbarten Gehäuseteil befestigte Vorsprünge eingreifen und einrasten. Diese Verbindungsart ist bevorzugt, da die benötigten Auskerbungen und Vorsprünge bereits mit in die entsprechenden Gehäuseteile integriert werden können, so dass keine zusätzlichen Bauteile benötigt werden. Zudem kann eine solche Verbindung sehr schnell und einfach durch Zusammendrücken der Gehäuseteile hergestellt werden. Obwohl reversible Verbindungen zwischen den Gehäuseteilen denkbar sind, werden die Gehäuseteile bevorzugt derartig miteinander verbunden, dass ein zerstörungsfreies Öffnen des Gehäuses nicht möglich ist. Diese Maßnahme beugt insbesondere Verletzungen durch herumliegende Einzelteile wie zum Beispiel die nadelbespickten Walzen vor.

Die erfindungsgemäße Vorrichtung kann ferner Halte- und Führungsmittel aufweisen, um den Transport und Gebrauch der Vorrichtung sicherer zu gestalten. So kann zum Beispiel ein Knauf auf der Gehäuseoberseite angebracht sein. Ferner ist es denkbar, die Gehäuseoberfläche mit einer Struktur wie Noppen oder Rillen zu versehen, um ein Abrutschen der Führungshand zu erschweren. Als besonders geeignet hat sich in diesem Zusammenhang jedoch ein am Gehäuse angebrachter Bügel erwiesen. Die Vorrichtung kann an dem Bügel problemlos angehoben und transportiert werden. Ferner kann der Bügel beim Gebrauch der Vorrichtung effektiv ein unbeabsichtigtes Herunterfallen der Vorrichtung verhindern. Üblicherweise ist der Bügel dabei über die Oberseite des Gehäuses verschwenkbar, so dass die Bedienperson wählen kann, ob der Bügel aufgerichtet ist oder nicht. Dabei sind sowohl Anordnungen möglich, bei denen der Bügel über die gesamte Oberseite des Gehäuses verschwenkt werden kann, als auch Anordnungen, die nur eine teilweise Verschwenkbarkeit des Bügels zulassen. Zweckmäßig ist der Bügel so am Gehäuse angebracht, dass die Schwenkrichtung des Bügels in Rollrichtung der Walzen orientiert ist und der Bügel somit quer zur Rollrichtung stehen kann. Im aufgerichteten Zustand des Bügels, im Folgenden Arbeitsposition genannt, kann die Bedienperson mit der Hand in den Bügel hingreifen, so dass die Oberseite der Vorrichtung die Handinnenfläche berührt und der Bügel die Handaußenfläche der Bedienhand umspannt. Dazu ist der Bügel entsprechend dimensioniert. In der herabgeklappten Position des Bügels, im Folgenden Lagerposition genannt, wird der Bügel quer zur Axialrichtung der Walzen bewegt. Bevorzugt fügt sich der Bügel dabei an das Gehäuse an, so dass die Vorrichtung auch in der Lagerposition des Bügels von einer Hand festgehalten werden kann. Ferner ist die Vorrichtung nunmehr in ihren äußeren Abmessungen bedeutend kleiner, womit eine platzsparende Lagerung ermöglicht wird.

Eine Befestigung des Bügels an der erfindungsgemäßen Vorrichtung erfolgt auf übliche Art und Weise. Ist der Bügel nicht schwenkbar, so können Bügel und Gehäuse aus einem Teil gefertigt sein. Ferner kann ein solcher Bügel auch angeklebt oder angeschweißt werden. Ebenso ist es denkbar, dass der Bügel Rastverbindungen oder Ösen aufweist, die über das Gehäuse geschoben werden und anschließend mit am Gehäuse angebrachten entsprechenden Aufnahmeelementen einen Formschluss herzustellen. Bevorzugt ist der Bügel aber schwenkbar mit der erfindungsgemäßen Vorrichtung verbunden, um den Bügel durch Aufrichten auf schnelle und einfache Weise verfügbar zu machen und umgekehrt. Dazu können zum Beispiel Bolzen, Verschraubungen etc. eine Verbindung zwischen Bügel und Gehäuse herstellen. Um die für die erfindungsgemäße Vorrichtung benötigte Anzahl an Einzelteilen zu senken, ist es allerdings erstrebenswert, einen Schwenkmechanismus zu verwenden, der keine zusätzlichen Bauteile zur Fertigung der Vorrichtung benötigt. Dazu werden Ausstülpungen, typischerweise in Form von Halbkugel, Kalotten oder Zylindern, am Bügel oder Gehäuse angebracht, welche von korrespondierenden Einstülpungen oder Lochungen aufgenommen werden, die am jeweils gegenüberliegenden Bauteil angebracht sind. Diese spezielle Art der Befestigung ermöglicht darüber hinaus, den Bügel reversibel am Gehäuse anzubringen, bzw. nach Belieben durch leichtes Auseinanderbiegen der Seitenteile des Bügels ohne dauerhafte Beschädigung auch wieder zu entfernen.

Zur Aufbewahrung einer erfindungsgemäßen Vorrichtung kann ferner ein spezieller Transportbehälter zur Verfügung gestellt werden. Dieser weist einen Innenraum auf, der die Vorrichtung ganz oder zumindest teilweise aufnehmen kann. In einer besonders bevorzugten Ausführung weist der Transportbehälter Stützen auf, die auf dem Boden und/oder im unteren Bereich der Seitenwände angebracht sind. Beim Einbringen der Vorrichtung in den Transportbehälter liegt die Vorrichtung mit der Unterkante des Gehäuses auf den Stützen auf, wobei die Auflagefläche der Stützen zweckmäßig mindestens so weit von der Bodenfläche des Transportbehälters beabstandet ist, dass die von den Walzen radial nach außen vorstehenden Nadeln den Boden des Transportbehälters nicht berühren. Einerseits werden die Nadeln nunmehr vollständig von abschirmenden Teilen umgeben, und ein ungewolltes Berühren der Nadeln durch Personal etc. wird unterbunden. Andererseits werden die Nadelspitzen geschont, da kein direkter Kontakt der Nadelspitzen zu einem harten Untergrund besteht. In einer weiteren Ausführungsform dient der Transportbehälter zusätzlich als Reinigungsbad für die Vorrichtung. Dazu wird er mit entsprechenden Reinigungs- und Sterilisationsflüssigkeiten wie zum Beispiel 3% Wasserstoffperoxid-Lösung oder 70% medizinischem Alkohol befüllt.

Um den Transportbehälter abzudecken, ist ferner ein passender Deckel vorgesehen. Neben der Funktion als Verschmutzungsschutz bei längerer Lagerung der Vorrichtung kann dieser insbesondere den Verdunstungsverlust der verwendeten Reinigungs- und Sterilisationsflüssigkeiten eindämmen.

Neben der bereits erwähnten bevorzugten Verwendung von Edelstahl für die Herstellung der Nadeln und der Walzenachsen können die übrigen Bestandteile der erfindungsgemäßen Vorrichtung jeweils aus geeigneten Materialien gefertigt werden. Vorzugsweise wird dazu ein Kunststoff und insbesondere ein Kunststoff, welcher im Spritzgussverfahren verarbeitet werden kann, eingesetzt. Darüber hinaus ist es aber auch denkbar, dass Einzelteile zum Beispiel aus Metall und insbesondere aus Edelstahl bestehen. Die verwendeten Kunststoffe, Metalle und ausgehärteten Klebstoffe sollten vorzugsweise dermatologisch unbedenklich sein. Weiterhin sollten die verwendeten Klebstoffe kompatibel mit den entsprechenden Kunststoffen sein. Dies könnte exemplarisch Cyanacrylat-Klebstoff sein. Ferner sind nahezu beliebige Kombinationen von unterschiedlichen geeigneten Materialien denkbar. Es ist allerdings bevorzugt, dass die verwendeten Materialien beständig gegenüber den üblicherweise zur Sterilisation und Reinigung verwendeten Bedingungen sind. Dies umfasst beispielsweise sowohl Sterilisationsmethoden mit UV- und Gammastrahlen als auch gebräuchliche Reinigungs- und Sterilisationsflüssigkeiten wie wässrige Wasserstoffperoxidlösungen und alkoholische Verdünnungen.

Die Erfindung soll nachfolgend anhand der Zeichnungen näher erläutert werden. Darin zeigen die Fig. 1a bis 6 im Einzelnen:
- Fig. 1a: eine schematische seitliche Durchsicht einer bevorzugten Ausführungsform der erfindungsgemäßen Vorrichtung,
- Fig. 1b: eine schematische frontale Durchsicht der in Fig. 1a dargestellten Ausführungsform,
- Fig. 1c: eine Darstellung der Vorrichtung gemäß Fig. 1a in einer Betrachtung von der Hautseite her,
- Fig. 2a: eine schematische Darstellung einer auf einem Strebenpaar gelagerten einzelnen Walze,
- Fig. 2b: eine schematische Darstellung einer bevorzugten Ausbildung der Achsen senkrecht zur Längsachse der Achse,
- Fig. 2c: eine Seitenansicht auf eine Strebe mit Fixiermittel,
- Fig. 3a: eine Draufsicht auf eine exemplarische Scheibenoberfläche einer mit Nadeln bestückten Scheibe,
- Fig. 3b: eine rückwärtige Ansicht auf die in Fig. 3a gezeigte Scheibe,
- Fig. 3c: eine Darstellung der zum Inneren einer Walze weisenden Oberfläche einer Endscheibe,
- Fig. 4a: eine Seitenansicht auf eine in einer Strebe gelagerten Walze mit über alle Scheiben durchgehenden Reihen von Nadeln,
- Fig. 4b: eine Frontalansicht auf die in Fig. 4a gezeigten Walze,
- Fig. 5a: eine Draufsicht auf die Unterseite eines zweiteiligen Gehäuses,
- Fig. 5b: einen Querschnitt entlang der Linie VII durch ein zweiteiliges Gehäuse,
- Fig. 6a: eine seitliche Durchsicht der Vorrichtung mit Bügel in Ruhe- und Transportposition,
- Fig. 6b: eine Rückansicht der Vorrichtung mit Bügel in Ruhe- und Transportposition,
- Fig. 7: eine schematische Darstellung der Vorrichtung in einem Transportbehälter,

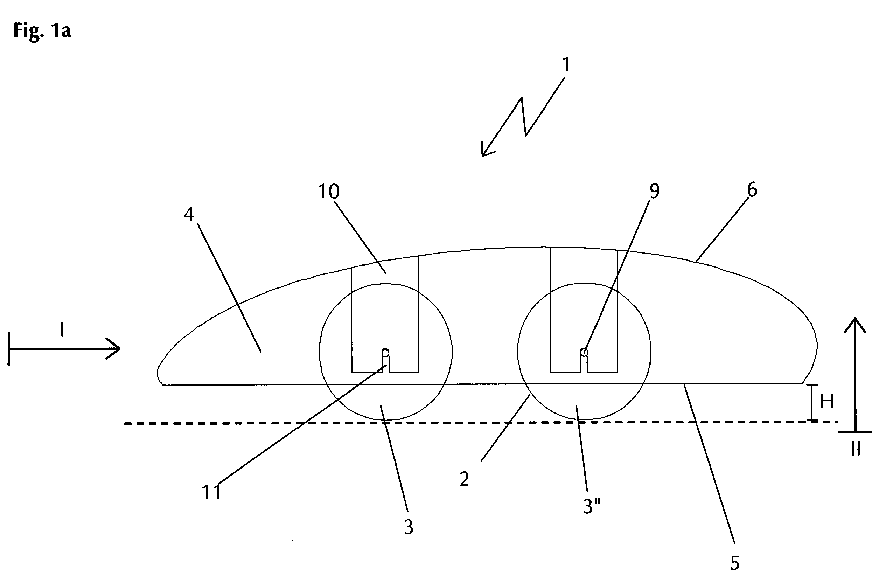

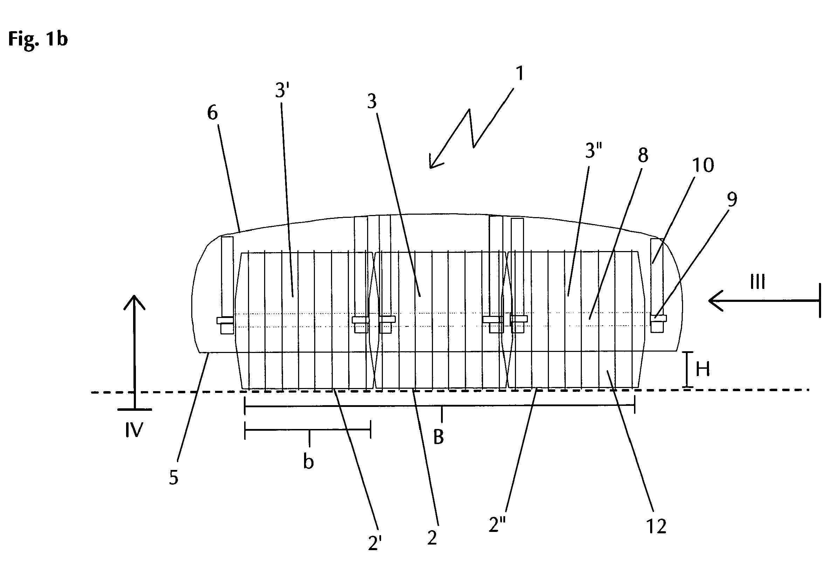

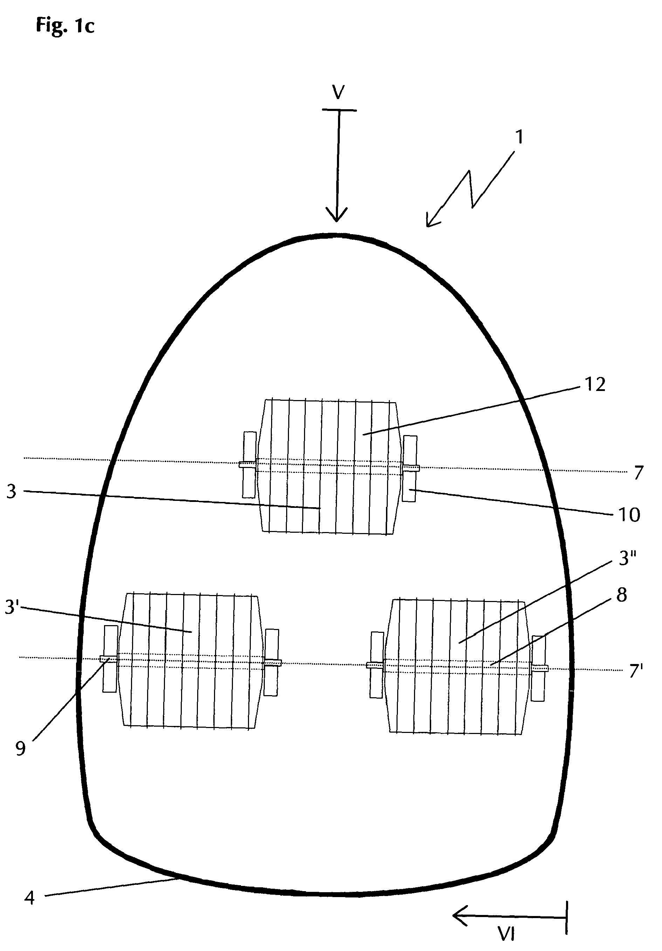

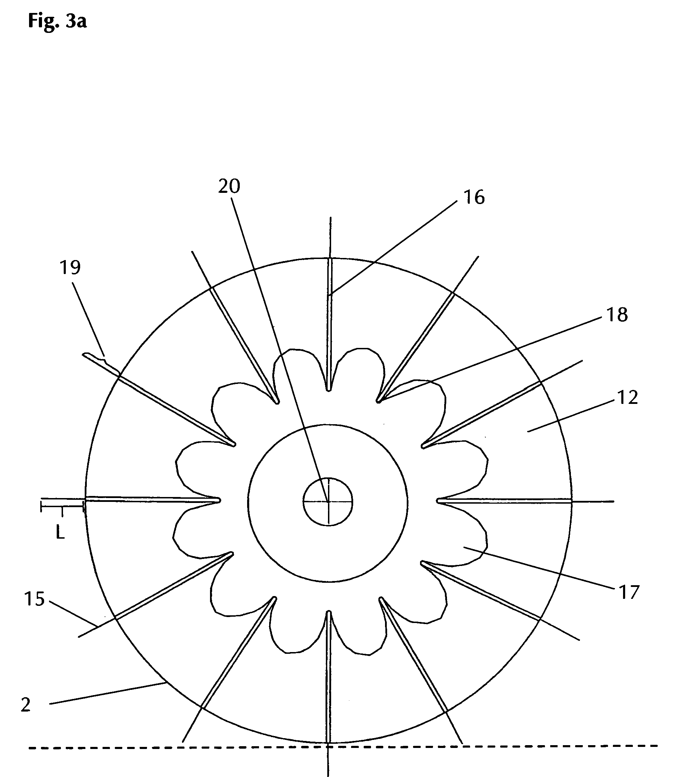

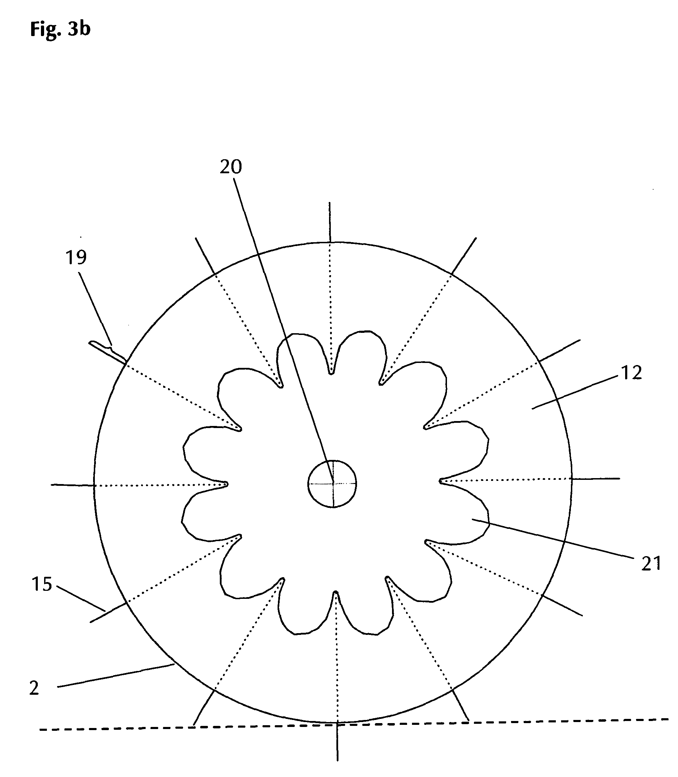

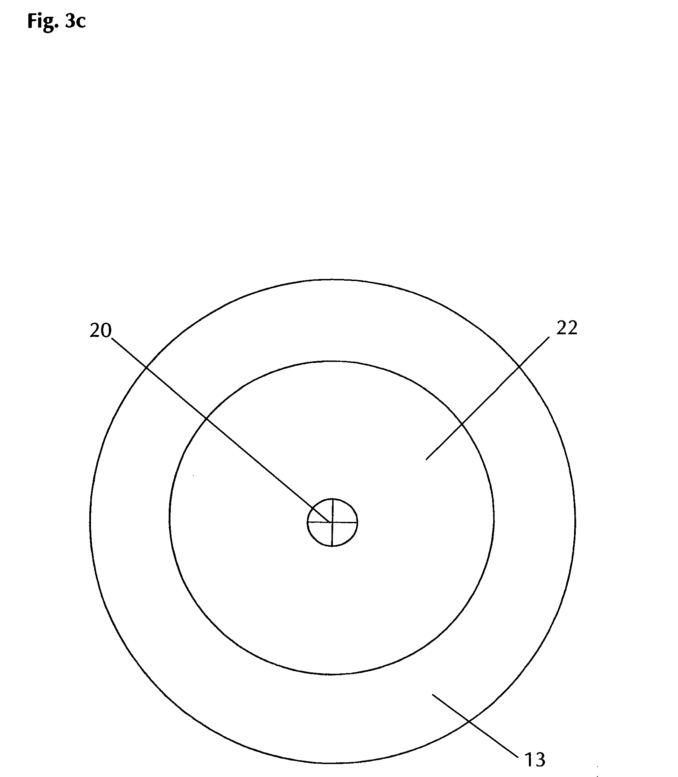

Eine besonders bevorzugte Ausbildung einer erfindungsgemäßen Vorrichtung 1 ist in den Fig. 1a, 1 bund 1c anhand schematischer Durchsichten dargestellt. Die mit römischen Zahlen beschrifteten Pfeile geben die Beziehungen der Fig. 1a (III und VI), 1b (I und V) und 1c (II und IV) untereinander an. Die über die Außenumfangsflächen 2, 2' und 2" der Walzen 3, 3' und 3" vorstehenden Nadelspitzen sind hier der Übersichtlichkeit halber nicht im Einzelnen dargestellt. Der detaillierte Aufbau der Walzen 3, 3' und 3" ist vielmehr den Fig. 3a, 3b und 3c zu entnehmen.

Die in den Fig. 1a, 1b und 1c dargestellte Vorrichtung 1 umfasst ein Gehäuse 4. Die Gehäuseunterseite 5 des Gehäuses 4 ist der zu behandelnden Hautoberfläche (als gestrichelte Linie in Fig. 1a und Fig. 1b dargestellt) zugewandt, die Gehäuseoberseite 6 ist entsprechend der zu behandelnden Hautoberfläche abgewandt. Ferner weist die Vorrichtung 1 die drei Walzen 3, 3' und 3" auf, die über die Gehäuseunterseite 5 mit einer Höhe H von 5 mm hervorstehen. Die Gehäuseoberseite 6 schirmt die Walzen 3, 3' und 3" nach oben hin ab, so dass die Vorrichtung mit der Hand leicht geführt werden kann, ohne die Walzen 3, 3' und 3" unbeabsichtigt mit der Hand zu berühren. Die Walzen 3, 3' und 3" sind dabei derartig angeordnet, dass die Längsachse 7 der Walze 3 parallel zu der Längsachse 7', entlang derer die beiden Walzen 3' und 3" koaxial zueinander angeordnet sind, verläuft. Die Längsachsen 7 und 7' sind 3 cm voneinander beabstandet. Ferner ist die Walze 3 in Relation zu den Walzen 3' und 3" so am Gehäuse 4 angebracht, dass bei einer Projektion der Längsachse 7 auf die Längsachse 7' die Walze 3 zwischen den Walzen 3' und 3" liegt und die Walzen 3, 3' und 3" V-förmig zueinander angeordnet sind, und zwar so, dass sich die Einzelbreiten b der Unterkanten der Außenumfangsflächen 2, 2' und 2" einer jeden Walze 3, 3' und 3" zu einer zusammenhängenden Gesamtbreite B ergänzen. In der vorliegenden Vorrichtung 1 beträgt die Einzelbreite b 2 cm und die Gesamtbreite B entsprechend 6 cm. Im Vergleich zu einer Vorrichtung die nur eine einzige der beschriebenen Walzen 3, 3' und 3" aufweist, kann durch diese spezielle Anordnung der Walzen 3, 3' und 3" ein Hautareal mit einer definierten Fläche in einem Drittel der Zeit gleichmäßig abgerollt werden.

Jede Walze 3, 3' oder 3" durchläuft entlang ihrer Längsachse 7, bzw. 7' jeweils eine Achse 8. Die Achse 8 steht jeweils in Axialrichtung zu beiden Seiten der Walzen 3, 3' oder 3" mit den Achsenüberständen 9 über. Zur Befestigung der Walzen 3, 3' und 3" am Gehäuse 4 sind Streben 10 paarweise am Gehäuse 4 angebracht, die sich in Richtung der zu behandelnden Hautoberfläche erstrecken. Um die Achsenüberstände 9 der Achsen 8 aufnehmen zu können, ist an jeder Strebe 10 auf der der Hautoberfläche zugewandten Seite eine Aussparung 11 angebracht, die einen Achsenüberstand 9 der Achse 8 aufnimmt.

Fig. 2a ist eine Ausschnittsvergrößerung im Bereich zweier am Gehäuse 4 angebrachter Streben 10, die die Walze 3 tragen. Dazu durchläuft die Achse 8 die Walze 3 entlang ihrer Längsachse 7. Im Einzelnen ist die Walze aus sieben aneinandergereihten Scheiben 12 aufgebaut, an die sich in Axialrichtung zu beiden Seiten die Endscheiben 13 anschließen. Zwischen den einzelnen Scheiben 12 und den Endscheiben 13 sind die Nadeln (hier nicht dargestellt) gelagert und stehen über die Außenumfangsfläche 2 der Walze 3 hervor. Der detaillierte Aufbau der einzelnen Scheiben 12 und der Endscheiben 13 ist den Fig. 3a, 3b und 3c zu entnehmen.

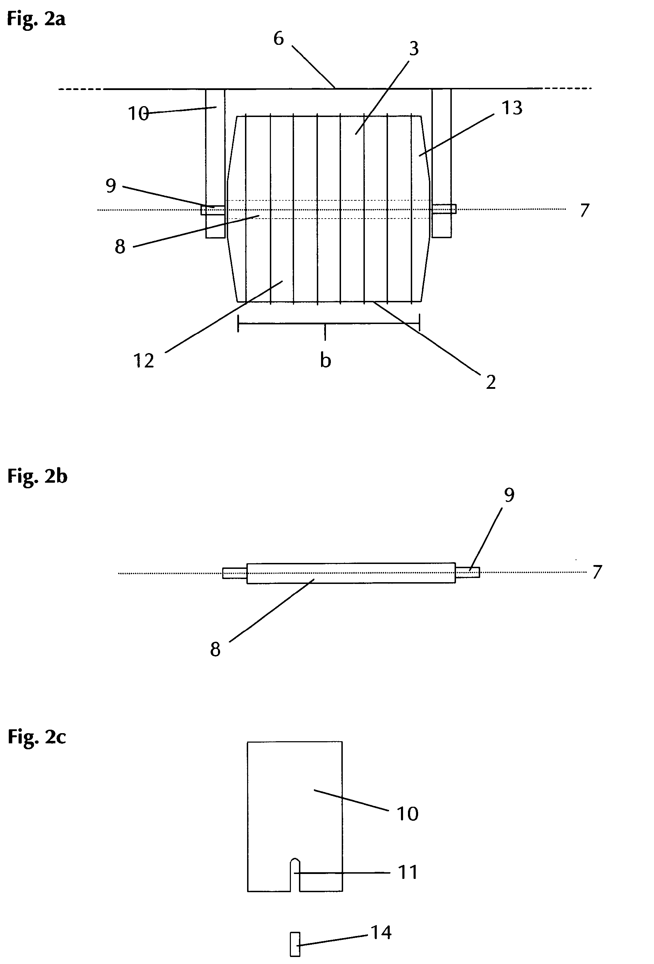

Die Walzen 3, 3' und 3" werden entlang ihrer jeweiligen Längsachse 7 oder 7' von der Achse 8 durchlaufen, deren bevorzugte Ausbildung in Fig. 2b dargestellt ist. Die Achse 8 weist an ihren axialen Enden die Achsenüberstände 9 auf, die mit einem Durchmesser von 1 mm schmaler sind als der Mittelteil der Achse 8, der einen Durchmesser von 2 mm hat. Diese besondere Anordnung, dass die Achsenüberstände 9 einen geringeren Achsendurchmesser als die übrige Achse 8 haben, verhindert ein seitliches Herausrutschen der Achsen 8 aus den Aussparungen 11 der Streben 10, da die Aussparung 11 der in Figur 2c gezeigten Strebe 10 eine Breite haben, die im Wesentlichen dem Durchmesser der Achsenüberstände 9 entspricht. Die Achse 8 kann daher lediglich im Bereich des Achsenüberstandes 9 in die Aussparung 11 geführt werden. Die abgebildete Strebe 10 ist in ihren Dimensionen so ausgelegt, dass sie die beim Führen der Vorrichtung 1 auf der Hautoberfläche resultierenden Kräfte aufnehmen kann und eine sichere und nachhaltige Positionierung der Walze 3 ermöglicht. Dazu ist die Strebe 10 in Axialrichtung der Walzen 2 mm dick und hat eine Breite senkrecht zur Axialrichtung der Walzen von 10 mm.

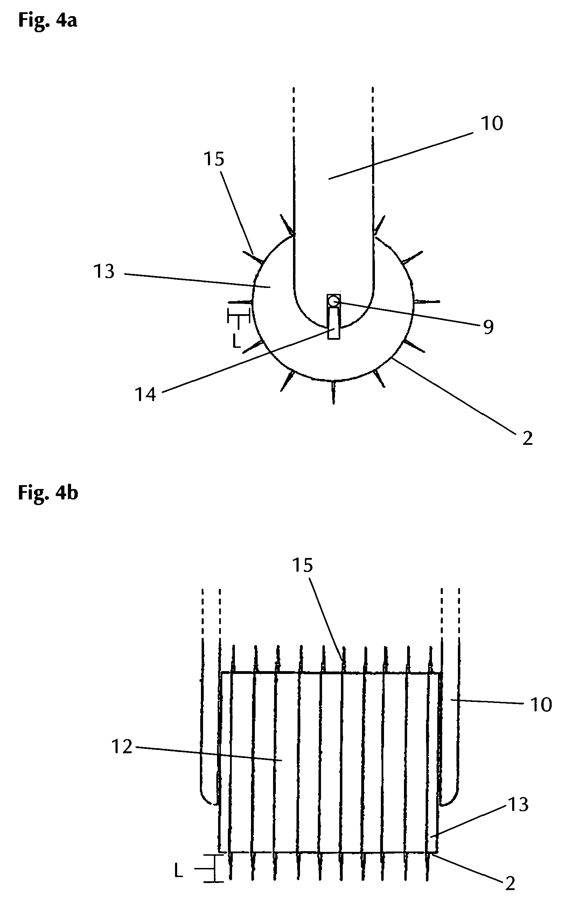

Um ein Herausgleiten der Achsenüberstände 9 aus den Aussparungen 11 in Richtung der Hautoberfläche wie zum Beispiel beim Transport der Vorrichtung zu verhindern, dient ein Fixiermittel 14, dass in Fig. 2c dargestellt ist. Die Breite des Fixiermittels 14 ist so angelegt, dass es sich gerade in die Aussparung 11 einfügt und so die Aussparung 11 in Richtung der Hautoberfläche verschließt. Nach dem Einlegen der Achsenüberstände 9 wird das Fixiermittel 14 in die Aussparung 11 geschoben und dort zum Beispiel eingeklebt (vgl. Fig. 4a).

Insbesondere in Fig. 1b ist der gestapelte Aufbau der gleichartig aufgebauten Walzen 3, 3' und 3" aus einzelnen aneinandergereihten Scheiben 12 zu erkennen. Dieser besondere Aufbau ermöglicht die gleichmäßige Verteilung der Nadeln in parallelen Reihen sowohl über Einzelwalzen, wie zum Beispiel Walze 3, als auch über die Gesamtheit der Walzen 3, 3' und 3".

Fig. 3a zeigt ein Beispiel einer mit Nadeln 15 bestückten Scheibe 12 in Draufsicht auf eine der Scheibenoberflächen. Die Scheibe 12 ist aus Kunststoff und durch Spritzgießen hergestellt. Auf der Oberfläche der Scheibe 12 sind mehrere Vertiefungen 16 vorgesehen, welche sich strahlenförmig vom Scheibeninneren bis zu deren äußeren Rand hin erstrecken. Der vom Scheibenmittelpunkt aus gemessene Winkel zwischen benachbarten Vertiefungen 16 beträgt jeweils 30°. In jede der Vertiefungen 16 ist eine Nadel 15 eingelegt und mit einem Klebstoff befestigt. Im Zentrum der Scheibe 12 befindet sich ein zahnkranzähnlicher Zentriervorsprung 17, welcher über die restliche Oberfläche der Scheibe 12 vorsteht. Die Mulden zwischen den einzelnen Zähnen des Zahnkranzes bilden gleichzeitig einen Anschlag 18 für die Nadeln 15, deren hintere Enden in den Mulden liegen. Die Anschläge 18 verhindern, dass die Nadeln 15 unter Druck in das Innere der Scheibe 12 verschoben werden können.

Alle Nadeln 15 weisen im Wesentlichen eine einheitliche Länge auf. Sie stehen mit der Länge L über die Außenumfangsflächen 2, 2' und 2" der Walzen 3, 3' und 3" bzw. jeder einzelnen der Scheiben 12 vor. Dabei laufen die Nadeln 15 nach außen spitz zu. In diesem Spitzenbereich 19 weisen die Nadeln einen Durchmesser von 0,08 mm auf. Ferner entspricht die Länge L der gewünschten Eindringtiefe der Nadeln in die zu behandelnde Haut. In der Fig. 3a ist dies mit der gestrichelten Linie im unteren Bereich der Zeichnung dargestellt. Diese Linie stellt schematisch die Hautoberfläche dar. Beim Drehen der Walze bewegt sich diese über die Hautoberfläche vorwärts, und die Nadeln 15 dringen nacheinander in die Hautoberfläche ein.

Um eine Achse durch eine Walze, respektive durch die Scheiben 12, führen zu können, weist jede der Scheiben 12 eine Durchgangsbohrung 20 im Scheibenzentrum auf. Durch diese wird die in Fig. 2b dargestellte Achse 8 geschoben und anschließend in den Aussparungen 11 eines Strebenpaares 10 gelagert.

Fig. 3b zeigt die Scheibe 12 aus Fig. 3a von der rückwärtigen Seite. Anstelle des Zahnkranzförmigen Zentriervorsprungs 17 weist diese Seite der Scheibe 12 eine entsprechend geformte Zentriervertiefung 21 auf. Werden mehrere Scheiben 12, wie zum Beispiel in Fig. 2a dargestellt, zu einer Walze 3 vereinigt, kommt der Zentriervorsprung 17 in der Zentriervertiefung 21 einer benachbarten Scheibe 12 zu liegen. Dadurch wird nicht nur sichergestellt, dass die benachbarten Scheiben exakt zueinander zentriert werden, sondern es wird auch dafür gesorgt, dass die Nadeln 15 benachbarter Scheiben in einer bestimmten Orientierung zueinander zu liegen kommen. Dabei ist es denkbar, die Nadeln 15 benachbarter Scheiben in Reihen anzuordnen, welche parallel zu den Achsen 8 der einzelnen Walzen 3 verlaufen. Es ist jedoch ebenso gut möglich, Nadeln 15 benachbarter Scheiben gestaffelt zueinander anzuordnen.

Bei der in Fig. 2a dargestellten Einzelwalze sind sieben der in Fig. 3a gezeigten Scheiben 12 parallel zueinander angeordnet. Die stirnseitigen Abschlüsse der Walze 3 bilden die Endscheiben 13, welche sich in ihrer Ausbildung von den Mittelscheiben 12 unterscheiden. Beispielsweise sind die nach außen weisenden Oberflächen der Endscheiben 13 glatt und weisen keine Vertiefungen oder Vorsprünge auf. Die Endscheiben 13 unterscheiden sich auch untereinander. Eine der Endscheiben weist eine innenseitige Oberflächengestaltung auf, welche derjenigen entspricht, die in Fig. 3a dargestellt ist. Der zahnkranzförmige Zentriervorsprung 17 greift formschlüssig in die entsprechende Zentriervertiefung 21 der benachbarten Mittelscheibe 12 ein. Außerdem sind die Vertiefungen 16 mit Nadeln 15 bestückt und bilden die achte Nadelreihe der in der in Fig. 2a dargestellten Walze. Insgesamt weist diese Walze damit 96 Nadeln auf.

Die zweite Endscheibe 13 ist in Fig. 3c dargestellt. Fig. 3c zeigt die zum Inneren der Walze 3 weisende Oberfläche. Im Bereich um die Durchgangsöffnung 20 besitzt die Endscheibe 13 eine Vertiefung 22. Diese Vertiefung 22 ist gerade groß genug, um den Zahnkranz-Vorsprung 17 der benachbarten Mittelscheibe 12 aufnehmen zu können.

Die einzelnen Scheiben, welche die mit Nadeln 15 bestückte Walze 3 der erfindungsgemäßen Vorrichtung 1 bilden, sind mittels eines Klebstoffs aneinander befestigt. Zweckmäßig wird hier derselbe Klebstoff verwendet, der auch zum Einkleben der Nadeln 15 in den Vertiefungen 16 auf den Scheiben 12 dient.

Durch den besonderen Aufbau der Walzen aus aneinandergereihten Scheiben 12 können die Nadeln, wie es in den Fig. 4a und 4b dargestellt ist, in über alle Scheiben durchgehenden Reihen von Nadeln angeordnet werden. Alternativ ist aber auch ein seitlicher Versatz der Nadeln 15 mit gestaffelter Anordnung zueinander möglich. Der Deutlichkeit halber wurden in den Fig. 4a und 4b die Proportionen der Nadeln erheblich vergrößert.

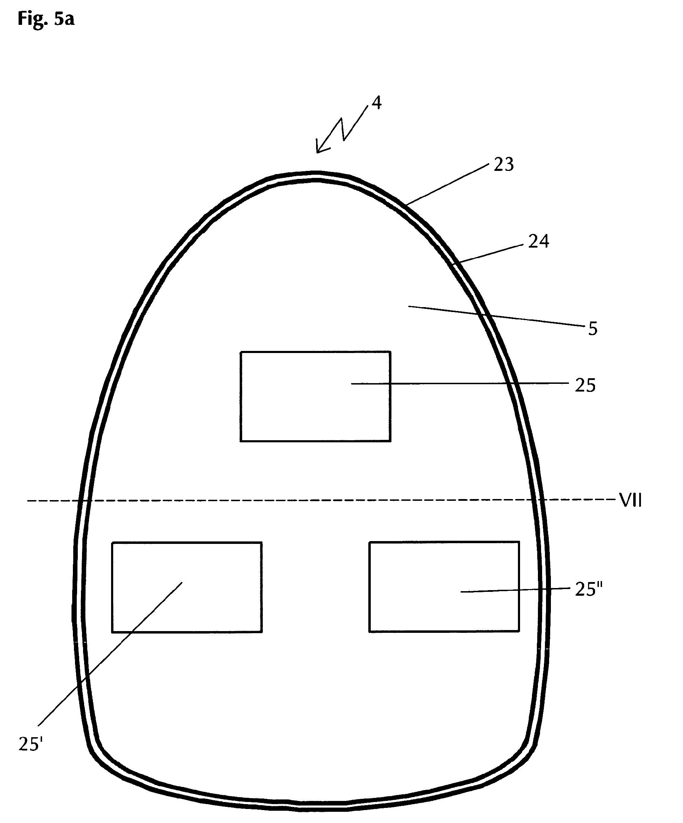

Fig. 5a zeigt eine Draufsicht auf die Unterseite 5 des Gehäuses 4. Das dargestellte Gehäuse 4 besteht aus einem Oberteil 23 und einem Unterteil 24. Das Unterteil weist auf seiner Fläche drei rechteckige Öffnungen 25, 25' und 25" auf, durch die die Walzen 3, 3' und 3" (hier nicht dargestellt) hindurchragen können. Dabei sind die Öffnungen 25, 25' und 25" so angelegt, dass der Freiraum zwischen ihnen und den hindurchragenden Walzen 3, 3' und 3" möglichst klein ist.

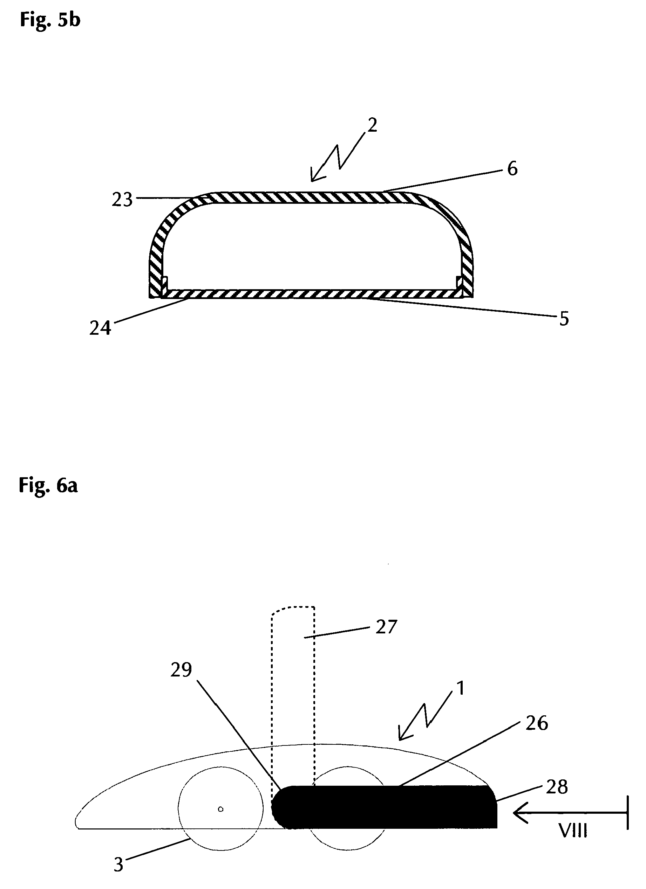

Zur Verbindung des Oberteils 23 mit dem Unterteil 24 umgreift das Oberteil 23 mit seinen Seitenwänden die Seitenwände des Unterteils 24. In Fig. 5b ist dazu ein Querschnitt des Gehäuses 2 entlang der gestrichelten Linie VII aus Fig. 5a dargestellt. Ober- und Unterteil können beispielsweise durch Rastverbindungen miteinander verbunden sein.

Um den Transport und eine sichere Führung der Vorrichtung 1 zu erleichtern ist ein Bügel 26 am Gehäuse 4 angebracht (vgl. seitliche Durchsicht der Fig. 6a). Der Bügel 26 kann über die Gehäuseoberseite 6 verschwenkt werden und zwischen einer hochgeklappten Arbeitsposition 27 und der herabgeschwenkten Lagerposition 28 frei hin- und herbewegt werden. Die Befestigung des Bügels 26 erfolgt hier über jeweils an den Bügelenden befindliche Erhebungen 29, die in am Gehäuse 4 angebrachte runde Lochungen eingeführt werden. Dabei stehen die Erhebungen 29 mit einer Höhe von 5 mm von der Bügelinnenfläche ab. In der gezeigten Ausführung besitzt der Bügel 26 eine ausreichende Elastizität, so dass der Bügels 26 reversibel auseinandergebogen werden kann. Vorteilhaft ist an dieser speziellen Anordnung, dass der Bügel 26 beliebig häufig vom Gehäuse 4 entfernt und wieder angebracht werden kann.

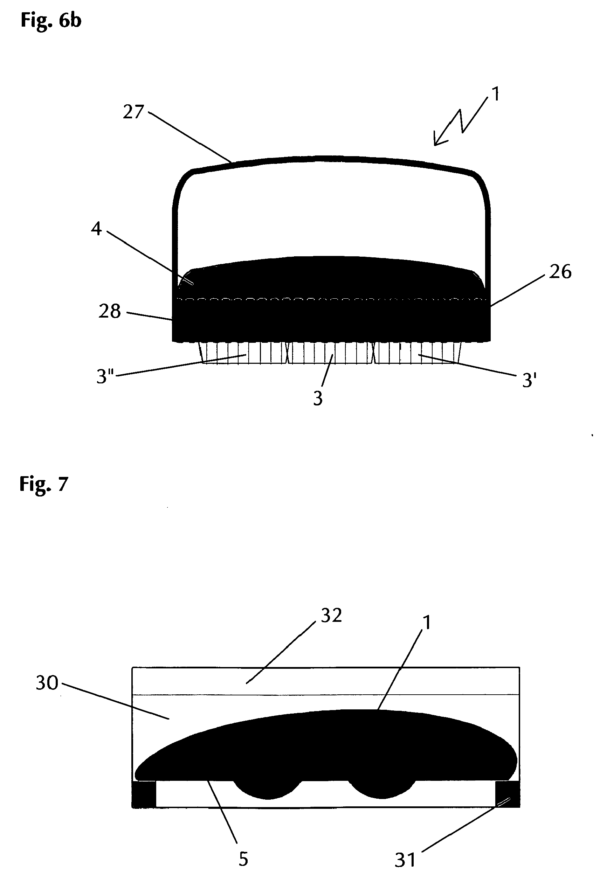

Fig. 6b zeigt die in Fig. 6a beschriebene Anordnung in einer Rückansicht entlang des Pfeils VIII aus Fig. 6a.

In der schematischen Seitenansicht in Fig. 7 ist ein Transportbehälter 30 dargestellt, in den die Vorrichtung 1 eingefügt ist. Dabei sind entlang der Kanten zwischen den Seitenwänden und dem Behälterboden des Transportbehälters 30 Stützelemente 31 angebracht, auf denen die Unterseite 5 des Gehäuses 4 zu liegen kommt. Ferner ist für den Transportbehälter 30 ein passender Deckel 32 vorgesehen, um den Transportbehälter 30 nach oben hin verschließen zu können. Neben der reinen Transportfunktion kann der Transportbehälter 30 zusätzlich als Reinigungsbad durch die Aufnahme klassischer Reinigungs- und Desinfektionsflüssigkeiten wie zum Beispiel alkoholischer Lösungen und verdünnter Wasserstoffperoxid-Lösungen etc. genutzt werden. Zweckmäßig wird der Behälter dazu aus einem Material hergestellt, das stabil gegenüber derartigen Reinigungs- und Desinfektionsflüssigkeiten ist. Dies ist vorzugsweise ein geeigneter Kunststoff.

## Patentansprüche

1. Vorrichtung (1) zum Einbringen eines Wirkstoffes in die Haut mit einer um ihre Längsachse (7) drehbar gelagerten Walze (3), auf deren Außenumfangsfläche (2) eine Vielzahl von Nadeln (15), die zumindest in einem über die Außenumfangsfläche (2) vorstehenden Spitzenbereich (19) nach außen spitz zulaufend ausgebildet sind, radial nach außen vorsteht,
**dadurch gekennzeichnet,**
**dass** mindestens zwei mit Nadeln (15) versehene Walzen (3, 3') parallel zueinander und seitlich derart gegeneinander versetzt angeordnet sind, dass bei einer Projektion einer der Walzen (3) in der Weise, dass ihre senkrecht zur Achsrichtung projizierte Längsachse (7) auf der Längsachse (7') der parallel angeordneten Walze (3') zu liegen kommt, die Bereiche der Außenumfangsflächen (2, 2') beider Walzen (3, 3'), in denen die Nadeln (15) nach außen vorstehen, sich in Axialrichtung nicht überschneiden.

2. Vorrichtung gemäß Anspruch 1,
**dadurch gekennzeichnet,**
**dass** mehr als zwei, vorzugsweise drei, Walzen vorhanden sind, wobei jede weitere Walze (3") parallel zu einer in einer Parallelrichtung zu den Längsachsen unmittelbar folgenden Walze (3') angeordnet ist.

3. Vorrichtung gemäß Anspruch 2,
**dadurch gekennzeichnet,**
**dass** jede weitere Walze (3") koaxial zu einer in einer Parallelrichtung zu den Längsachsen (7) übernächsten Walze (3) angeordnet ist.

4. Vorrichtung gemäß einem der vorhergehenden Ansprüche,
**dadurch gekennzeichnet,**
**dass** die Walzen (3, 3', 3") baugleich sind.

5. Vorrichtung gemäß einem der vorhergehenden Ansprüche,
**dadurch gekennzeichnet,**
**dass** die Nadeln (15) gleichmäßig über die Außenumfangsflächen (2, 2') aller Walzen (3, 3') und zudem gleichmäßig über die Gesamtbreite (B) der Walzen (3, 3') verteilt sind.

6. Vorrichtung gemäß einem der vorhergehenden Ansprüche,
**dadurch gekennzeichnet,**
**dass** die Nadeln (15) mit gleicher Länge (L) und insbesondere mit einer Länge von 0,12 bis 1,5 mm über die Außenumfangsflächen (2, 2') vorstehen.

7. Vorrichtung gemäß einem der vorhergehenden Ansprüche,
**dadurch gekennzeichnet,**
**dass** die Nadeln (15) im Spitzenbereich (19) einen Durchmesser von 0,05 bis 0,1 mm, insbesondere von 0,08 mm, aufweisen.

8. Vorrichtung gemäß einem der vorhergehenden Ansprüche,
**dadurch gekennzeichnet,**
**dass** der Durchmesser der Nadeln (15) außerhalb des Spitzenbereiches (19) zwischen 0,15 und 0,3 mm liegt.

9. Vorrichtung gemäß einem der vorhergehenden Ansprüche,
**dadurch gekennzeichnet,**
**dass** die Walzen (3, 3') aus parallelen Scheiben (12, 12', 12") bestehen, zwischen denen die Nadeln (15) eingebettet sind, und dass die Walzen (3, 3') eine die Längsachse (7, 7') umfassende Durchgangsbohrung (20) aufweisen, in welche eine Achse (8, 8') eingesteckt ist.

10. Vorrichtung gemäß Anspruch 9,
**dadurch gekennzeichnet,**
**dass** auf einer der Scheibenoberflächen wenigstens ein Zentriervorsprung (17) vorgesehen ist, welcher in eine entsprechende Zentriervertiefung (21) in der Oberfläche einer benachbarten Scheibe (12) eingreift, wobei der Zentriervorsprung (17) gleichzeitig als Anschlag (18) für wenigstens eine der Nadeln (15) dient und insbesondere die Form eines im Zentrum der Scheibe (12) vorspringenden Zahnkranzes aufweist.

11. Vorrichtung nach einem der vorhergehenden Ansprüche,
**dadurch gekennzeichnet,**
**dass** die Vorrichtung (1) ein die Walzen (3, 3') tragendes und auf der hautabgewandten Seite (6) abschirmendes Gehäuse (4) umfasst.

12. Vorrichtung gemäß Anspruch 11,
**dadurch gekennzeichnet,**
**dass** das Gehäuse (4) in Richtung auf die hautzugewandte Seite (5) vorstehende, paarweise angeordnete und zueinander beabstandete Streben (10) aufweist, wobei die Streben (10) eines Strebenpaares (10, 10') auf der hautzugewandten Stirnseite Aussparungen (11) aufweisen, die die aus einer Walze (3, 3') axial herausragenden Achsenabschnitte (9) der Walze (3, 3') aufnehmen.

13. Vorrichtung gemäß Anspruch 11 oder 12,
**dadurch gekennzeichnet,**
**dass** die Walzen (3, 3') so am Gehäuse (4) befestigt sind, dass zumindest der Spitzenbereich (19) der Nadeln (15) über eine durch die Unterkante des Gehäuses (4) aufgespannte Ebene vorsteht.

14. Vorrichtung gemäß Anspruch 12 oder 13,
**dadurch gekennzeichnet,**
**dass** Fixiermittel (14) vorhanden sind, um die überstehenden Achsenabschnitte (9) der Walze (3, 3') in den Aussparungen (11) der Streben (10) von der hautzugewandten Seite her zu sichern.

15. Vorrichtung gemäß einem der Ansprüche 11 bis 14,
**dadurch gekennzeichnet,**
**dass** das Gehäuse (4) aus einem Oberteil (23) und einem Unterteil (24) besteht, wobei das Unterteil (24) Öffnungen (25, 25') für die Walzen (3, 3') aufweist.

16. Vorrichtung gemäß Anspruch 15,
**dadurch gekennzeichnet,**
**dass** die Streben (10) am Oberteil (23) und die Fixiermittel (14) am Unterteil (24) befestigt sind.

17. Vorrichtung gemäß einem der Ansprüche 11 bis 16,
**dadurch gekennzeichnet,**
**dass** sie einen quer zur Rollrichtung der Walzen (3, 3') stehenden, über die Oberseite (6) des Gehäuses (4) schwenkbaren Bügel (26) umfasst.

18. Vorrichtung gemäß einem der Ansprüche 11 bis 17,
**dadurch gekennzeichnet,**
**dass** das Gehäuse (4) aus Kunststoff und insbesondere aus einem Spritzguss-Kunststoff besteht.
